# EUROPÄISCHE PATENTSCHRIFT

(11) **EP 1 214 398 B1**
(45) Veröffentlichungstag und Bekanntmachung des Hinweises auf die Patenterteilung: **23.04.2008**
(21) Anmeldenummer: 00916745.3
(22) Anmeldetag: 10.02.2000
(51) Int. Cl.: C12N 7/04, C12N 15/00

(54) **CHIMÄRE VIRUS-ÄHNLICHE PARTIKEL BZW. CHIMÄRE CAPSOMERE VON BPV (BOVINE PAPILLOMA VIRUS)**
CHIMERIC VIRUS-LIKE PARTICLES OR CHIMERIC CAPSOMERS FROM BPV
PARTICULES CHIMERIQUES ANALOGUES A DES VIRUS ET CAPSOMERES CHIMERIQUES DE PAPILLOMAVIRUS BOVIN

(30) Priorität: 11.02.1999 DE 19905883
(43) Veröffentlichungstag der Anmeldung: 19.06.2002
(73) Patentinhaber: Deutsches Krebsforschungszentrum Stiftung des öffentlichen Rechts, 69120 Heidelberg (DE)
(72) Erfinder: GISSMANN, Lutz, D-69168 Wiesloch (DE); MÜLLER, Martin, D-69118 Heidelberg (DE); MÜLLER, Hermann, D-04155 Leipzig (DE)
(74) Vertreter: Schüssler, Andrea
(86) Internationale Anmeldenummer: PCT/DE2000/000426
(87) Internationale Veröffentlichungsnummer: WO 2000/047722

(56) Entgegenhaltungen:
- EP-A- 0 133 123
- WO-A-96/26277
- WO-A-99/02694
- WO-A-99/18220
- US-A- 5 744 142
- MÜLLER M ET AL: "Chimeric papillomavirus-like particles" VIROLOGY,US,ACADEMIC PRESS,ORLANDO, Bd. 234, 21. Juli 1997 (1997-07-21), Seiten 93-111, XP002091857 ISSN: 0042-6822
- POTTER H.L. ET AL.: "Nucleotide sequence of bovine Papillomavirus Type 2 late region" JOURNAL OF GENERAL VIROLOGY, Bd. 66, 1985, Seiten 187-193, XP000923183
- CHEN E.Y. ET AL.: "The primary structure and genetic organization of the bovine papilomavirus type 1 genome" NATURE, Bd. 299, 1982, Seiten 529-534, XP000915049
- GROFF D.E. ET AL.: "E6 Protein Bovine papillomavirus type 2" EMBL DATABASE, 1. Juli 1989 (1989-07-01), XP002156901 HEIDELBERG, DE
- GROFF D.E. ET AL.: "Probable E7 Protein. Bovine papillomavirus type 2" EMBL DATABASE, 1. Juli 1989 (1989-07-01), XP002156902 HEIDELBERG, DE

## Beschreibung

Die vorliegende Erfindung betrifft bestimmte chimäre Virus-ähnliche Partikel (cVLPs) bzw. chimäre Capsomere (cCs) von BPV, Fusionsproteine und sie kodierende DNAs sowie die Verwendung der cVLPs bzw. der cCs zur Immunisierung von Huftieren, insbesondere Pferden.

Bei Huftieren, insbesondere Pferden, treten häufig Sarkoide auf. Dies sind exophytisch wachsende Hauttumoren, die an verschiedenen Körperteilen lokalisiert sein können. Auch weisen Sarkoide oftmals Sequenzen von Bovinen Papillomviren (BPVs) auf. Sarkoide werden in der Regel chirurgisch entfernt, was jedoch aufgrund ihrer Lokalisation, z.B. am Auge oder Ohr, oftmals schwierig ist. Ferner weisen Sarkoide eine hohe Rezidivrate auf, wodurch sie ein erhebliches Problem für Tierärze, Züchter und Halter darstellen.

Der vorliegenden Erfindung liegt somit die Aufgabe zugrunde ein Mittel bereitzustellen, mit dem gegen Sarkoide vorgegangen werden kann, wobei vorstehende Nachteile vermieden werden.

Erfindungsgemäß wird dies durch die Gegenstände in den Patentansprüchen erreicht.

Die vorliegende Erfindung beruht auf den Erkenntnissen des Anmelders, daß sich cVLPs bzw. cCs von BPV, wobei letztere unvollständige cVLPs sind, eignen gegen Sarkoide von Huftieren, insbesondere Pferden, prophylaktisch und therapeutisch vorzugehen. Solche cVLPs bzw. cCs enthalten Fusionsproteine, die Fragmente von L1- oder L2-Proteinen von BPV und frühe Proteine von BPV, z.B. E6- oder E7-Proteine, bzw. Fragmente davon umfassen. Er hat erkannt, daß durch die cVLPs bzw. cCs Antikörper induziert bzw. zytotoxische T-Zellen stimuliert werden können, die gegen BPV gerichtet sind. Ferner hat-er erkannt, daß durch die Antikörper eine Infektion mit BPV verhindert und durch die zytotoxischen T-Zellen eine Abstoßung der Sarkoide und der von BPV infizierten Zellen erreicht werden kann.

Erfindungsgemäß werden die Erkenntnisse des Anmelders genutzt, bestimmte cVLPs bzw. cCs von BPV zur Immunisierung von Huftieren gegen Sarkoide zu verwenden, wobei die cVLPs bzw. cCs ein Fusionsprotein enthalten, das ein Fragment des L1- oder L2-Proteins von BPV und ein E6 oder E7 Protein von BPV bzw. ein Fragment davon umfaßt, wobei (a) das Fragment des L1-Proteins die Aminosäuresequenzen 1-469 bzw. 1-472 von SEQ ID NO: 2 oder 10 umfaßt, (b) das Fragment des E6-Proteins die Aminosäuresequenzen 1-55,47-101 oder 83-137 von SEQ ID NO: 3 oder 5 umfaßt, oder (c) das Fragment des E7-Proteins die Aminosäuresequenzen 1-54,46-100 oder 72-127 von SEQ ID NO:8 oder 12.

Der Ausdruck "BPV" umfaßt jegliche Typen von BPV, insbesondere die Typen BPV1 und BPV2.

Der Ausdruck "cVLPs bzw. cCs von BPV" umfaßt jegliche cVLPs bzw. cCs, die Fusionsproteine enthalten, welche die vorstehenden Fragmente von L1- oder L2-Proteinen von BPV und frühe Proteine von BPV, z.B. E6- oder E7-Proteins, bzw. die vorstehenden Fragmente davon umfassen. Die Bereitstellung von cVLPs bzw. cCs kann durch übliche Verfahren erfolgen. Beispielsweise können die für die einzelnen Fragmente bzw. Proteine kodierenden DNAs in einer Ligase-Reaktion miteinander verknüpft werden. Die erhaltenen DNA-Moleküle können in Zellen, z.B. E. coli, Hefe, Säugetier- oder Insektenzellen, exprimiert werden, wodurch Fusionsproteine erhalten werden, die sich zu cVLPs bzw. cCs falten bzw. zusammenlagern können. Günstig kann es sein, die für die Fusionsproteine kodierenden DNA-Möleküle in Gegenwart von Virus-DNA, z.B. Vaccinavirus- bzw. Baculovirus-DNA, zu exprimieren, wobei die Expression mit Baculovirus-DNA bevorzugt ist. Es wird auf die nachstehenden Beispiele verwiesen. Erhaltene cVLPs bzw. cCs können eine oder mehrere Arten von Fusionsproteinen enthalten, wobei bei mehreren Arten sich die Fusionsproteine sowohl in den Fragmenten der L1- oder L2-Proteine als auch der frühen Proteine bzw. deren Fragmenten unterscheiden können. Die Isolierung der cVLPs bzw. cCs kann durch übliche Verfahren, z.B. Dichtegradientenzentrifugation, erfolgen.

Der Ausdruck "Huftiere" umfaßt jegliche Art von Tieren, die Hufe tragen, insbesondere Pferde, Rinder und Wild.

Der Ausdruck "Immunisierung von Huftieren gegen Sarkoide" umfaßt jegliche Verfahren, mit denen in Huftieren durch vorstehende cVLPs bzw. cCs Antikörper induziert bzw. zytotoxische T-Zellen stimuliert werden können, die gegen BPV gerichtet sind. Günstig kann es sein, pro Tier ca. 100 µg - 1 mg der cVLPs bzw. cCs zu injizieren. Ferner kann es günstig sein, nach ca. 14 Tagen eine "Booster-Injektion" mit ca. gleicher Menge durchzuführen, wobei es besonders günstig sein kann, in den einzelnen Injektionen L1- oder L2-Fragmente von unterschiedlichen BPV-Typen zu verwenden. Der Nachweis von gegen BPV gerichteten Antikörpern bzw. zytotoxischen T-Zellen kann durch übliche Verfahren erfolgen. Beispielsweise wird auf Roden et al. Journal of Virology, (Nov. 1994), 7570-7574 bzw. Brostrom et al., Am J Vet Res. 7, (Juli 1996), 992-999 verwiesen.

Ein weiterer Gegenstand der vorliegenden Erfindung ist ein cVLP bzw. cC von BPV, das ein Fusionsprotein, wie vorstehend definiert, enthält.

Ein weiterer Gegenstand der vorliegenden Erfindung ist ein Fusionsprotein, das in vorstehendem cVLP bzw. cC enthalten ist. Für dieses Fusionsprotein gelten die vorstehenden Ausführungen entsprechend.

Ein weiterer Gegenstand der vorliegenden Erfindung ist eine DNA, die für ein vorstehendes Fusionsprotein kodiert, wobei die DNA eine Kombination von SEQ ID NO: 1 oder 11 und SEQ ID NO: 4, 6, 7 oder 9 oder eine degeneriete DNA Sequenz davon umfasst.

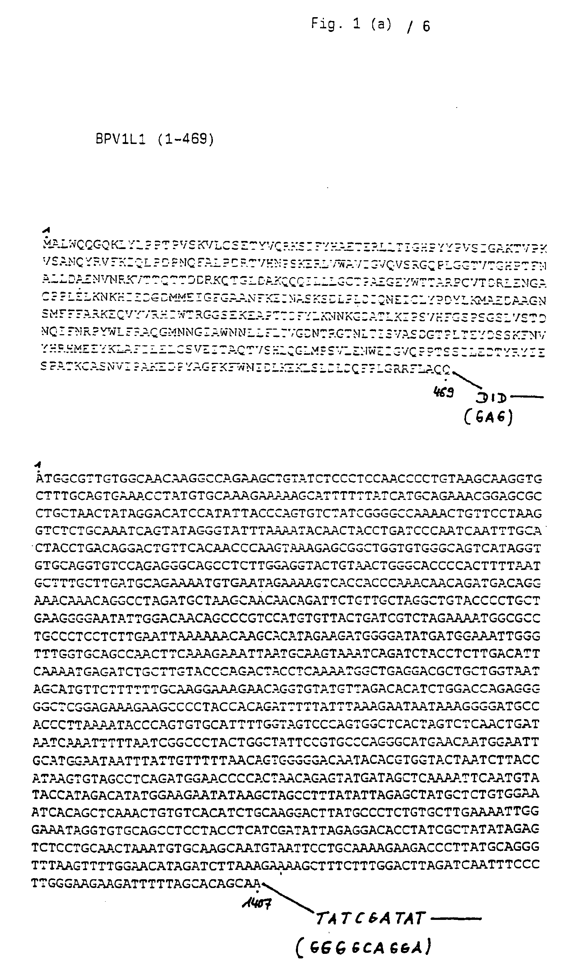

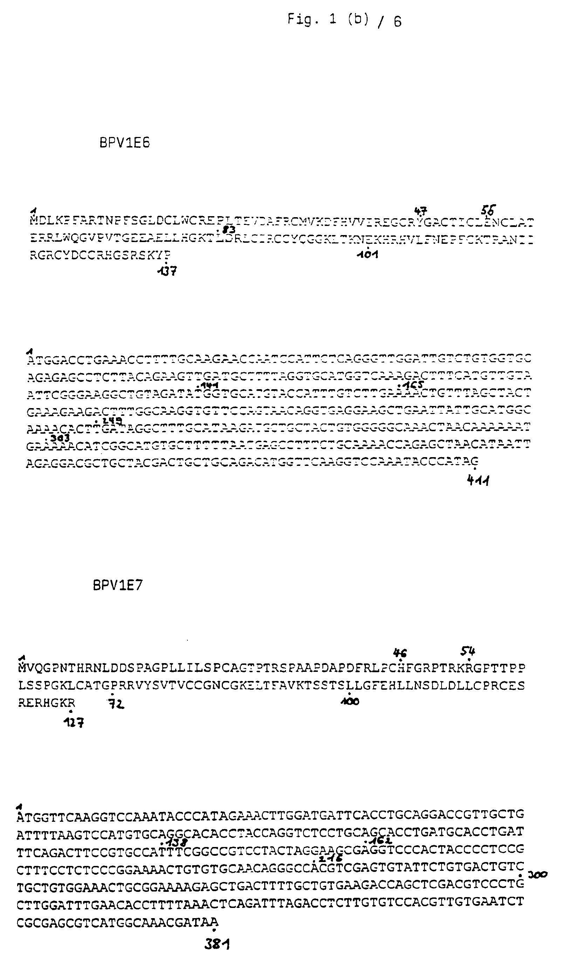

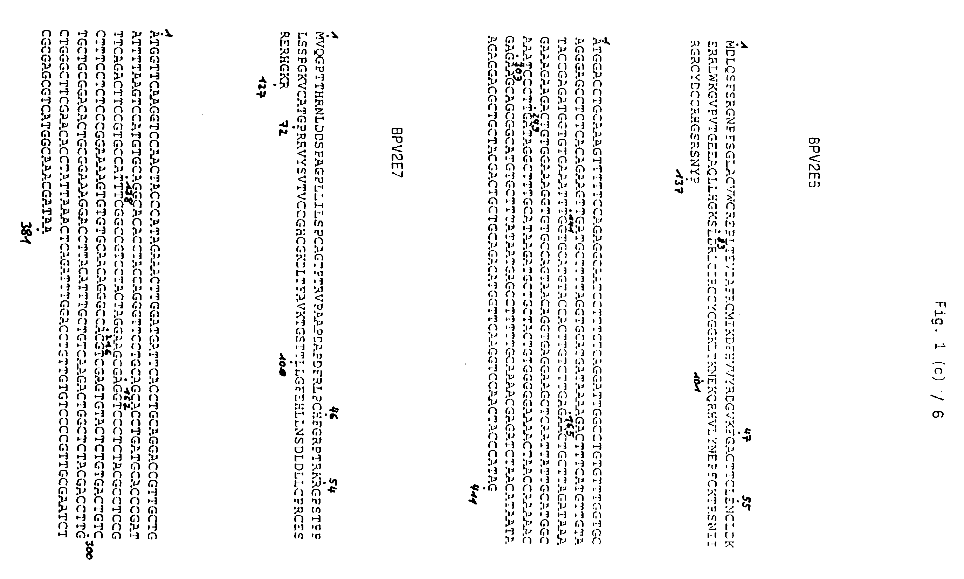

Eine bevorzugte DNA von Fig. 1, die für BPV1L1 (1-469) und BPVE7₁₋₁₂₇ bzw. BPVE7₁₋₅₄ kodiert, wurde als pVL1393 - BPV1L1/E7₁₋₁₂₇ bzw. pVL1393 - BPV1L1/E7₁₋₅₄ bei der DSMZ (Deutsche Sammlung von Mikroorganismen und Zellkulturen) unter DSM 12628 bzw. 12627 am 14. Jan. 1999 hinterlegt.

Eine erfindungsgemäße DNA kann als solche oder in Kombination mit jeglicher anderen DNA vorliegen. Insbesondere kann eine erfindungsgemäße in einem Expressionsvektor vorliegen. Beispiele solcher sind dem Fachmann bekannt. Im Falle eines Expressionsvektors für E. coli sind dies z.B. pGEMEX, pUC-Derivate, pGEX-2T, pET3b und pQE-8. Für die Expression in Hefe sind z.B. pY100 und Ycpadl zu nennen, während für die Expression in Säugetierzellen z.B. pKCR, pEFBOS, cDM8 und pCEV4 anzugeben sind. Für die Expression in Insektenzellen eignen sich besonders die Expressionsvektoren pAcSGHisNT-A und pVL 1393.

Der Fachmann kennt geeignete Zellen, um die erfindungsgemäße, in einem Expressionsvektor vorliegende DNA zu exprimieren. Beispiele solcher Zellen umfassen die E.coli-Stämme HB101, DH1, x1776, JM101, JM 109, BL21, SG 13009 und DH5α, den Hefe-Stamm Saccharomyces cerevisiae und die Säugetierzellen L, NIH 3T3, FM3A, CHO, COS, Vero und HeLa sowie die Insektenzellen sf9.

Desweiteren kennt der Fachmann Bedingungen transformiert bzw. transfizierte Zellen zu kultivieren. Auch sind ihm Verfahren bekannt das durch die erfindungsgemäße DNA exprimierte Fusionsprotein zu isolieren und zu reinigen. Ferner sind ihm Verfahren bekannt die durch die Faltung bzw. Zusammenlagerung des Fusionsproteins erhaltenen VLPs zu isolieren und zu reinigen.

Mit der vorliegenden Erfindung ist es möglich Huftiere, insbesondere Pferde, gegen Sarkoide zu immunisieren. Die Immunisierung umfaßt sowohl die Induzierung von Antikörpern als auch die Stimulierung von zytotoxischen T-Zellen, die gegen BPV gerichtet sind. Somit ist ein prophylaktisches und therapeutisches Vorgehen gegen Sarkoide von Huftieren, insbesondere Pferden, möglich.

### Kurze Beschreibung der Figuren:

- **Fig. 1**: zeigt die Aminosäure- und DNA-Sequenzen von erfindungsgemäßen Fusionsproteinen, die ein Fragment des L1-Proteins von BPV1, nämlich 1-469 bzw. 1-472, und BPV1/2 E6- bzw. E7-Proteine bzw. Fragmente davon umfassen. Das L1-Fragment von BPV1 (1-469) weist am C-Terminus die zusätzlichen Aminosäuren DID (470-472; TATCGATAT) auf. Diese stammen nicht aus dem L1-Protein, sondern von der in die DNA eingefügten Restriktionsschnittstelle EcoRV/ClaI. Das L1-Fragment von BPV1 (1-472) weist am C-Terminus die natürlichen Aminosäuren GAG (470-472; GGGGCAGGA) auf. Die E6-Fragmente von BPV1/2 umfassen die Aminosäuresequenzen 1-55, 47-101 bzw. 83-137. Die E7-Fragmente von BPV1/2 umfassen die Aminosäuresequenzen 1-54, 46-100 bzw. 72-127. Die Fusionsproteine können in erfindungsgemäßen cVLPs bzw. cCs vorliegen.

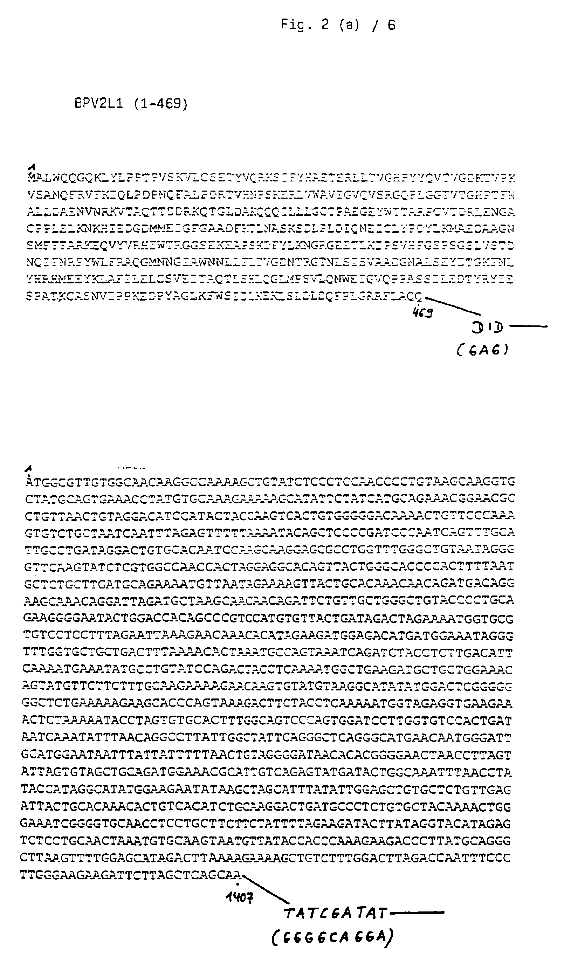

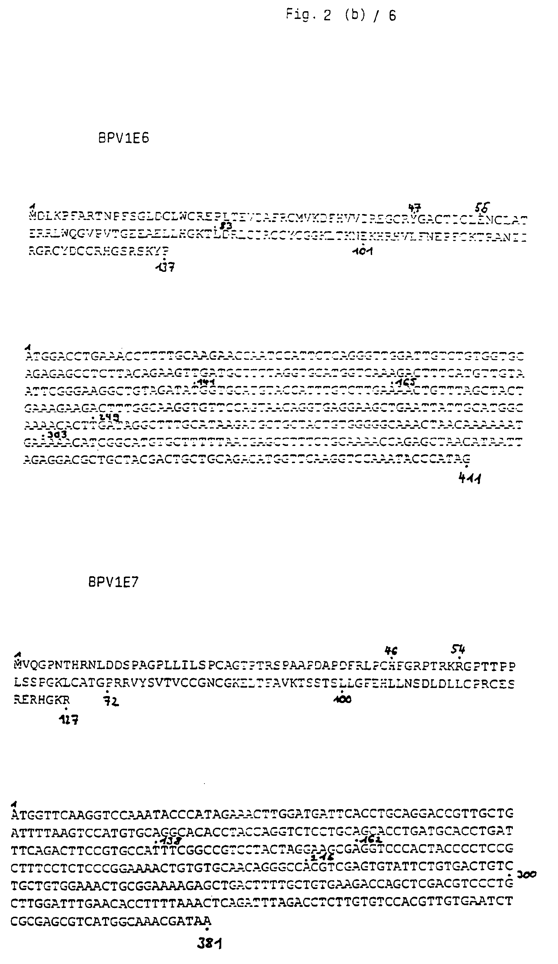

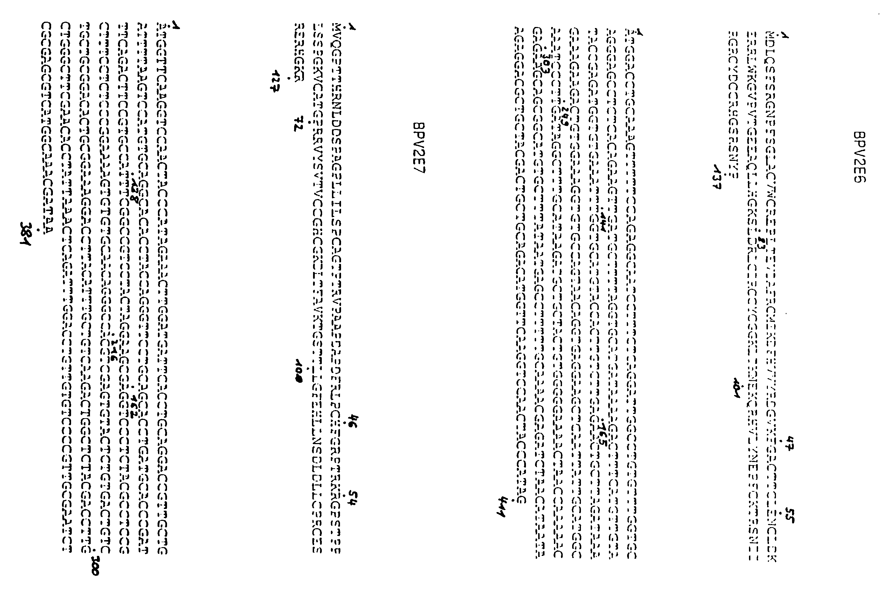

- Fig. 2: zeigt die Aminosäure- und DNA-Sequenzen von erfindungsgemäßen Fusionsproteinen, die ein Fragment des L1-Proteins von BPV2, nämlich 1-469 bzw. 1-472, und BPV1/2 E6- bzw. E7-Proteine bzw. Fragmente davon umfassen. Das L1-Fragment von BPV2 (1-469) weist am C-Terminus die zusätzlichen Aminosäuren DID (470-472; TATCGATAT) auf. Diese stammen nicht aus dem L1-Protein, sondern von der in die DNA eingefügten Restriktionsschnittstelle EcoRV/ClaI. Das L1-Fragment von BPV2 (1-472) weist am C-Terminus die natürlichen Aminosäuren GAG (470-472; GGGGCAGGA) auf. Die E6-Fragmente von BPV1/2 umfassen die Aminosäuresequenzen 1-55, 47-101 bzw. 83-137. Die E7-Fragmente von BPV1/2 umfassen die Aminosäuresequenzen 1-54, 46-100 bzw. 72-127. Die Fusionsproteine können in erfindungsgemäßen cVLPs bzw. cCs vorliegen.

Die Erfindung wird durch die nachstehenden Beispiele erläutert.

### Beispiel 1: Herstellung von erfindungsgemäßen cVLPs bzw. cCs

### (A) Herstellung des Fragments 1-469 des L1-Proteins von BPV1

Es wird von einem für das L1-Protein von BPV1 kodierenden Expressionsplasmid, pUC19-BPV1-L1, ausgegangen. Dieses wird in eine PCR-Reaktion eingesetzt, in der die nachfolgenden Primer 1 und 2 verwendet werden, die sich mit ihren 5'-Enden auf dem L1-Gen von BPV1 gegenüberstehen. Beide Primer tragen am 5'-Ende eine kurze Spacer Sequenz (AAA) sowie eine EcoRV Spaltstelle (unterstrichen). Primer 2 enthält zusätzlich ein Stop Codon (TGA). Die L1-spezifischen Sequenzen sind fett gedruckt.

7015 6986
Primer 1. AAAGATATCTTGCTGTGCTAAAAATCTTCTTCCCAAGGG
7038 7066
Primer 2. AAAGATATCTGAGAAAACGAAGAATTAGCCAAAAAACTTCC

Erhaltene PCR-Produkte werden gereinigt, mit EcoRV gespalten und mit T4 Ligase zirkularisiert, bevor sie zur Transformation von E. coli DH5α Zellen verwendet werden. Es werden Klone erhalten, die auf die Anwesenheit einer EcoRV-Spaltstelle getestet werden, die weder im L1-Gen noch im Vektor vorliegt. Einer dieser Klone zeigt nach Sequenzierung, daß er für das Fragment 1-469 des L1-Proteins von BPV1 kodiert. Dieser Klon wird mit pUC BPV1-L1ΔC bezeichnet.

### (B) Herstellung von Fragmenten des E7-Proteins von BPV1

Es wird von einem für das E7-Protein von BPV1 kodierenden Expressionsplasmid, pUC19-BPV1-E7, ausgegangen. Dieses wird in eine PCR-Reaktion eingesetzt, in der die nachfolgenden Primer 3, 4 und 5 verwendet werden. Die Primer 3-5 enthalten am 5'-Ende einen Spacer (TTTT) und eine EcoRV Spaltstelle (unterstrichen). Primer 3 enthält zusätzlich die Sequenz GAT (unterstrichen kursiv), wodurch eine mit der EcoRV Spaltstelle überlappende Clal Spaltstelle (ATC GAT) generiert (zur Identifizierung des Inserts in sense Orientierung) und ein zusätzliches Asp eingefügt werden. Primer 5 enthält zusätzlich ein Stop Codon. Die E7-spezifischen Sequenzen sind fett gedruckt. Die Primer 3 und 4 werden verwendet, um das komplette Gen des E7-Proteins zu amplifizieren, während die Primer 3 und 5 zur Amplifikation des Fragments 1-54 des E7-Proteins eingesetzt werden.

474 Start
506
Primer 3: TTTTGATATCGATATGGTTCAAGTCCAAATACCCATAGAAAC

862
833
Primer 4: TTTTGATATCTTATCGTTTGCCATGACGCTCGCGAGATTC

Stop 640
610
Primer 5: TTTTGATATCTTATCGCTTCCTAGTAGGACGGCCGAAATGGCAC

Erhaltene PCR-Produkte werden gereinigt, mit EcoRV gespalten und über die EcoRV-Spaltstelle in den Expressionsvektor Bluescript inseriert. Nach Transformation von E. coli DH5α Zellen werden Klone erhalten, die auf die Anwesenheit zweier EcoRV-Stelle getestet werden, von denen keine im E7-Gen und nur eine im Vektor vorliegt. Zwei dieser Klone zeigen nach Sequenzierung, daß sie für das komplette E7-Protein bzw. für das Fragment 1-54 des E7-Proteins kodieren. Der erste Klon wird mit Bluescript BPV1-E7 und der zweite Klon mit Bluescript BPV1-E7₁₋₅₄ bezeichnet.

### (C) Herstellung von erfindungsgemäßen Fusionsproteinen

Aus den in (B) erhaltenen Klonen Bluescript BPV1-E7 und Bluescript BPV1-E7₁₋₅₄ werden nach Spaltung mit EcoRV die E7-Sequenzen mittels Agarose-Gelelektrophorese isoliert und in die EcoRV-Spaltstelle von pUCBPV1-L1ΔC von (A) inseriert. Nach Transformation von E. coli DH5α werden Klone erhalten und sequenziert. Zwei dieser Klone zeigen, daß sie für ein Fusionsprotein kodieren, welches das Fragment 1-469 des L1-Proteins und das E7-Protein bzw. das Fragment 1-54 des E7-Proteins umfaßt. Der erste Klon wird mit pUCBPV1L1ΔCBPVE7₁₋₁₂₇ und der zweite mit pUCBPV1L1ΔCBPVE7₁₋₅₄ bezeichnet.

### (D) Herstellung von erfindungsgemäßen cVLPs bzw. cCs

Die BPV-Sequenzen von pUCBPV1L1ΔCBPVE7 bzw. pUCBPV1L1ΔCBPVE7₁₋₅₄ werden vom Vektor durch Bam HI abgespalten und in die Bam HI-Spaltstelle des Transfervektors pVL 1393 (Invitrogen) inseriert. Nach Transformation von E.coli DH5α Zellen werden Klone erhalten. Zwei dieser Klone weisen die vorstehenden BPV-Sequenzen auf, wobei der eine Klon mit pBPV1L1ΔCBPVE7 und der andere Klon mit pVLBPV1L1ΔCBPVE7₁₋₅₄ bezeichnet wird. Die Klone werden zusammen mit linearisierter Baculovirus DNA (Baculo-Gold, Pharmingen) in SF9 Zellen mittels des Calziumphosphat-Präzipitationsverfahrens kotransfiziert. Es werden erfindungsgemäße cVLPs bzw. cCs erhalten, die nach einem Verfahren von Müller et al., Virology 234, 93-111, gereinigt werden. Diese enthalten die Fusionsproteine BPV1L1ΔCBPVE7₁₋₁₂₇ bzw. BPVL1ΔCBPVE7₁₋₅₄.

### Beispiel 2: Immunisierung von Pferden gegen Sarkoide

Es werden zwei gesunde und zwei an Sarkoiden operierte Pferde herangezogen. Diesen werden 100 µg - 1 mg der cVLPs bzw. cCs von (D) +/- Adjuvans injiziert. Nach 14 Tagen wird eine "Booster"-Injektion mit gleicher Menge der VLPs durchgeführt. Den Pferden wird zu verschiedenen Zeitpunkten Blut entnommen und hinsichtlich der Induzierung von Antikörpern bzw. der Stimulierung von zytotoxischen T-Zellen, die gegen BPV gerichtet sind, getestet. Hierzu werden die Verfahren in Roden et al. Journal of Virology, (Nov. 1994), 7570-7574 bzw. Brostrom et al., Am J Vet Res. 7, (Juli 1996), 992-999 beschriebenen Verfahren durchgeführt.

Es zeigt sich, daß in den gesunden Pferden sowohl eine Antikörper-Induzierung als auch eine Stimulierung von zytotoxischen T-Zellen, die gegen BPV gerichtet sind, erfolgt ist. Ferner zeigt sich, daß in den an Sarkoiden operierten Pferden die Bildung von Rezidiven im Vergleich zu Kontrollen mit Plazebos verhindert worden ist.

## Patentansprüche

1. Chimäre Virus-ähnliche Partikel (cVLPs) bzw. chimäre Capsomere (cCs) des Bovinen Papillomvirus (BPV), enthaltend ein Fusionsprotein, das die Aminosäuresequenz 1-469 oder 1-472 von SEQ ID NO: 2 oder 10 und die Aminosäuresequenzen 1-55, 47-101 oder 83-137 von SEQ ID NO: 3 oder 5 oder die Aminosäuresequenzen 1-54, 46-100 oder 72-127 von SEQ ID NO: 8 oder 12 umfaßt.

2. Fusionsprotein nach Anspruch 1.

3. DNA, kodierend für das Fusionsprotein nach Anspruch 2, wobei die DNA umfasst eine Kombination von SEQ ID NO: 1 oder 11 und SEQ ID NO: 4, 6, 7 oder 9, oder eine degenerierte DNA Sequenz davon.

4. Expressionsvektor, enthaltend die DNA nach Anspruch 3.

5. Verwendung von cVLPs bzw. cCs von BPV zur Herstellung eines Arzneimittels zur Immunisierung von Huftieren gegen Sarkoide, wobei die VLPs bzw. cCs ein Fusionsprotein enthalten, das ein Fragment des L1- oder L2-Proteins von BPV und ein E6 oder E7-Protein von BPV bzw. ein Fragment davon umfasst, wobei (a) das Fragment des L1-Proteins die Aminosäuresequenzen 1-469 bzw. 1-472 von SEQ ID NO: 2 oder 10 umfaßt und (b) das Fragment des E6-Proteins die Aminosäuresequenzen 1-55, 47-101 oder 83-137 von SEQ ID NO: 3 oder 5 umfaßt, oder (c) das Fragment des E7-Proteins die Aminosäuresequenzen 1-54, 46-100 oder 72-127 von SEQ ID NO:8 oder 12.

6. Verwendung nach Anspruch 5, wobei BPV der Typ BPV 1 oder BPV2 ist.

7. Verwendung nach Anspruch 5 oder 6, wobei die Immunisierung eine Prophylaxe und/oder Therapie von Sarkoiden umfaßt.

8. Verwendung nach einem der Ansprüche 5-7, wobei die Huftiere Pferde sind.

## Claims

1. Chimeric virus-like particles (cVLPs) or chimeric capsomers (cCs) from bovine papilloma virus (BPV), which contain a fusion protein comprising the amino acid sequence 1-469 or 1-472 of SEQ ID NO:2 or 10 and the amino acid sequences 1-55, 47-101 or 83-137 of SEQ ID NO: 3 or 5 or the amino acid sequences 1-54, 46-100 or 72-127 of SEQ ID NO: 8 or 12.

2. A fusion protein according to claim 1.

3. A DNA coding for the fusion protein according to claim 2, wherein the DNA contains a combination of SEQ ID NO:1 or 11 and SEQ ID NO: 4, 6, 7 or 9 or a degenerated DNA sequence thereof.

4. An expression vector containing the DNA according to claim 3.

5. Use of cVLPs or cCs from BPV for producing a drug for immunizing ungulates against sarcoids, wherein the VLPs or cCs contain a fusion protein which comprises a fragment of the L1 or L2 proteins from BPV and a E6 or E7 protein from BPV or a fragment thereof, wherein (a) the fragment of the L1 protein comprises the amino acid sequences 1-469 or 1-472 of SEQ ID NO: 2 or 10 and (b) the fragment of the E6 protein comprises the amino acid sequences 1-55, 47-101 or 83-137 of SEQ ID NO: 3 or 5 or (c) the fragment of the E7 protein comprises the amino acid sequences 1-54, 46-100 or 72-127 of SEQ ID NO: 8 or 12.

6. Use according to claims 5, wherein the BPV is the BPV1

7. Use according to any one of claims 5 or 6, wherein the immunization comprises a prophylaxis and/or therapy of sarcoids.

8. Use according to any one of claims 5 to 7, wherein the ungulates are horses.

## Revendications

1. Particules chimères semblables à des virus (cVLPs) ou bien des capsomères chimères (cCS) du papillomvirus bovin (BPV), renfermant une protéine de fusion qui englobe les séquences d'acides aminés 1-469 ou 1-472 de SEQ ID N°: 2 ou 10 et les séquences d'acides aminés 1-55, 47-101 ou 83-137 de SEQ ID N° : 3 ou 5, ou bien les séquences d'acides aminés 1-54, 46-100 ou 72-127 de SEQ ID N° : 8 ou 12.

2. Protéine de fusion selon la revendication 1.

3. ADN codant de la protéine de fusion selon la revendication 2, **caractérisé en ce que** l'ADN englobe une combinaison de SEQ ID-N° : 1 ou 11 et SEQ ID N° : 4, 6, 7 ou 9 ou bien une séquence ADN dégénérée de celui-ci.

4. Vecteur d'expression renfermant l'ADN selon la revendication 3.

5. Utilisation de cVLPs ou cCs de BPV pour la préparation de médicaments d'immunisation d'ongulés contre les sarcoïdoses, **caractérisée en ce que** les VLPs ou les cCs contiennent une protéine de fusion qui comprend un fragment de la protéine L1 ou L2 de BPV et une protéine E6 ou E7 de BPV ou un fragment de celle-ci, et **en ce que** (a) le fragment de la protéine L1 comprend les séquences d'acides aminés 1-469 ou 1-472 de SEQ ID N° :2 ou 10, et (b) le fragment de la protéine E6 comprend les séquences d'acides aminés 1-55, 47-101 ou 83-137 de SEQ ID N° :3 ou 5, ou (c) le fragment de la protéine E7 des séquences d'acides aminés 1-54, 46-100 ou 72-127 de SEQ ID N° :8 ou 12.

6. Utilisation selon la revendication 5, **caractérisée en ce que** BPV est du type BPV1 ou BPV2.

7. Utilisation selon la revendication 5 ou 6, **caractérisée en ce que** l'immunisation comprend une prophylaxie et/ou une thérapie des sarcoïdoses.

8. Utilisation selon l'une des revendications 5 à 7, **caractérisée en ce que** les ongulés sont des chevaux.
